# DEMANDE DE BREVET EUROPEEN

(11) **EP 4 792 783 A1**
(43) Date de publication de la demande: **19.08.2026**
(21) Numéro de dépôt: 26157665.6
(22) Date de dépôt: 10.02.2026
(51) Int. Cl.: A61F 2/28, A61F 2/40

(54) **PROTHÈSE DE RECONSTRUCTION HUMÉRALE**

(30) Priorité: 12.02.2025 FR 2501462
(71) Demandeur: FX Shoulder Solutions, 01440 Viriat (FR)
(72) Inventeur: BARCO, Raúl, 28043 Madrid (ES); DERANLOT, Julien, 75015 Paris (FR); FULLICK, Robert, Houston, 77005 (US); SIEGEL, Herrick, Alabama, 35223 (US); SRIKUMARAN, Umasuthan, Maryland, 21042 (US)
(74) Mandataire: LLR

(57) **Abrégé**

L'invention a pour objet une prothèse humérale (2) comprenant :
- une partie distale (4) comprenant des moyens de fixation à un humérus réséqué,
- une partie métaphysaire (24) comprenant des moyens de fixation d'un élément articulaire d'une articulation gléno-humérale,
une partie diaphysaire (22) configurée pour remplacer une partie d'humérus réséquée, s'étendant entre la partie distale (4) et la partie métaphysaire (24) et étant fixée à la partie distale (4) et à la partie métaphysaire (24).

## Description

### Domaine technique

L'invention concerne les prothèses de reconstruction humérale, notamment suite à une perte osseuse importante.

### État de la technique

Certains patients peuvent souffrir d'une perte osseuse plus ou moins importante au niveau huméral, par exemple dans le cadre de l'apparition d'une tumeur dans la partie proximale de l'humérus conduisant à la réalisation d'une résection osseuse.

Dans un tel cas de figure, il est nécessaire de remplacer la partie distale de l'humérus réséqué pour reconstruire l'humérus et reformer une articulation avec la glène.

L'invention a pour but de fournir une prothèse humérale permettant une reconstruction partielle de l'humérus avec une certaine flexibilité lors de la reconstruction de l'humérus, reconstruction stable et permettant à un patient de recouvrer une articulation gléno-humérale fonctionnelle tout en supprimant toute douleur.

### Résumé de l'invention

A cet effet l'invention a pour objet une prothèse humérale comprenant :
- une partie distale comprenant des moyens de fixation à un humérus réséqué,
- une partie métaphysaire comprenant des moyens de fixation d'un élément articulaire d'une articulation gléno-humérale, et
- une partie diaphysaire configurée pour remplacer une partie d'humérus réséquée, s'étendant entre la partie distale et la partie métaphysaire et étant fixée à la partie distale et à la partie métaphysaire.

Ainsi, on obtient une prothèse permettant une reconstruction humérale et ce quelle que soit la longueur humérale à reconstruire. En effet, la présence d'une partie diaphysaire indépendante des parties distale et métaphysaire permet d'adapter cette dernière au patient ayant besoin d'une reconstruction humérale.

La partie distale permet d'assurer une fixation de l'ensemble de la prothèse humérale à un humérus réséqué et ce de manière stable. Son indépendance vis-à-vis du reste de la prothèse humérale permet de choisir une partie distale adaptée au cas se présentant au chirurgien.

Enfin, la partie métaphysaire, également indépendante des autres parties de la prothèse humérale, peut être choisie en fonction du patient.

Suivant d'autres caractéristiques optionnelles de la prothèse humérale prises seules ou en combinaison :
- la partie distale comprend une tige comprenant des moyens de fixation à la partie diaphysaire et destinée à s'étendre à l'intérieur d'une cavité médullaire d'un humérus réséqué,
- la partie distale comprend une bride placée sur la tige et destinée à entourer au moins partiellement une extrémité d'un humérus réséqué,
- la bride et la tige comprennent des premiers moyens d'indexage de la bride sur la tige,
- la bride et la tige comprennent des orifices de verrouillage de la partie distale à un humérus, les orifices de verrouillage étant destinés à être alignés et traversés par une vis de verrouillage lors du montage de la partie distale sur un humérus réséqué,
- l'orifice de verrouillage de la tige est de forme oblong,
- le ou les orifices de verrouillage de la bride comprennent un filetage interrompu par des encoches,
- la tige comprend des orifices d'ancrage destinés à être envahis par un matériau osseux de l'humérus réséqué,
- la partie diaphysaire comprend des trous de suture,
- la partie diaphysaire comprend un premier orifice de montage sur la partie distale coopérant avec un premier plot de montage porté par la partie distale,
- l'orifice de montage et le plot de montage comprennent des deuxièmes moyens d'indexage de la partie diaphysaire sur la partie distale,
- la partie métaphysaire comprend un deuxième orifice de montage sur la partie diaphysaire coopérant avec un deuxième plot de montage porté par la partie diaphysaire,
- la partie métaphysaire est orientable en rotation sur la partie diaphysaire lors du montage de la partie métaphysaire sur la partie diaphysaire,
- la partie métaphysaire comprend une extrémité proximale plane configurée pour recevoir un élément articulaire d'une articulation gléno-humérale,
- l'extrémité proximale plane s'étend dans un plan incliné par rapport à un axe d'extension de la partie diaphysaire selon un angle compris entre 30° et 60°, de préférence compris entre 40° et 50°, de préférence sensiblement égal à 45°,
- Une vis de blocage s'étend depuis la partie métaphysaire jusqu'à la partie distale et verrouille les parties distale, diaphysaire et métaphysaire entre elles,
- La vis de blocage comprend une extrémité filetée vissée dans un orifice fileté ménagé au niveau de la partie distale, et
- La vis de blocage comprend une butée en appui contre un épaulement de la partie métaphysaire.

L'invention a également pour objet un kit d'implantation pour la conception d'une prothèse humérale selon l'invention, le kit comprenant :
- Plusieurs parties distales de longueur et/ou largeur différentes,
- Plusieurs parties métaphysaires de longueur et/ou largeur différentes, et
- Plusieurs parties diaphysaires de longueur et/ou largeur différentes.

Suivant d'autres caractéristiques optionnelles du kit d'implantation prises seules ou en combinaison :
- la bride et/ou la tige comprennent un nombre et/ou un positionnement différents d'orifices de verrouillage d'une partie distale à une autre,
- la bride est d'une longueur différente d'une partie distale à une autre,
- la tige est d'une longueur différente d'une partie distale à une autre,
- la tige comprend un nombre et/ou un positionnement différents d'orifices d'ancrage d'une partie distale à une autre,
- la partie diaphysaire comprend un nombre et/ou un positionnement différents de trous de suture d'une partie diaphysaire à une autre, et
- l'extrémité proximale plane s'étend dans un plan dont l'inclinaison par rapport à un axe d'extension de la partie diaphysaire différent d'une partie métaphysaire à une autre.

### Brève description des figures

L'invention sera mieux comprise à la lecture de la description qui va suivre donnée uniquement à titre d'exemple et faite en se référant aux dessins annexés dans lesquels :
La [Fig. 1] est une vue en perspective d'une prothèse humérale selon un premier mode de réalisation l'invention,
La [Fig. 2] est une vue éclatée d'une prothèse humérale selon le premier mode de réalisation l'invention,
La [Fig. 3] est une vue en coupe longitudinale d'une prothèse humérale selon le premier mode de réalisation l'invention,
La [Fig. 4] est une vue en perspective d'une partie distale selon le premier mode de réalisation l'invention,
La [Fig. 5] est une vue en coupe longitudinale d'une partie distale selon le premier mode de réalisation l'invention,
La [Fig. 6] est une vue en perspective de la portion supérieure d'une tige d'une partie distale selon le premier mode de réalisation l'invention,
La [Fig. 7] est une vue en perspective d'une bride d'une partie distale selon le premier mode de réalisation l'invention,
La [Fig. 8] est une vue en perspective d'une portion inférieure d'une bride d'une partie distale selon le premier mode de réalisation l'invention,
La [Fig. 9] est une vue est une vue en perspective d'une portion d'une partie distale selon le premier mode de réalisation l'invention,
La [Fig. 10] est une vue est une vue en perspective d'une prothèse humérale selon un deuxième mode de réalisation l'invention, et
La [Fig. 11] est une vue éclatée d'une prothèse humérale selon le deuxième mode de réalisation l'invention.

### Description détaillée

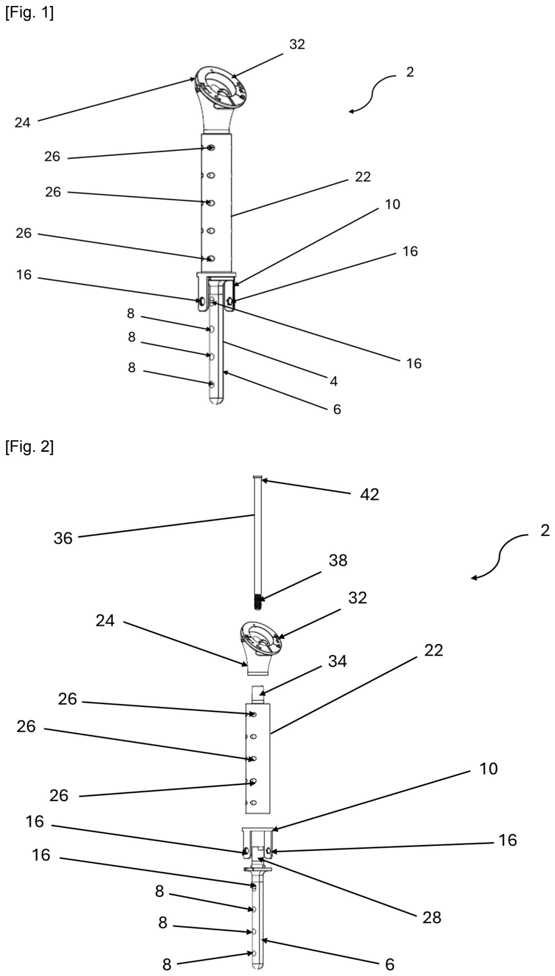

On se réfère désormais à la figure 1 illustrant un premier mode de réalisation d'une prothèse humérale 2. Comme expliqué ci-dessus, cette prothèse permet de reconstruire un humérus d'un patient ayant subi une résection plus ou moins importante de l'humérus, notamment suite à un cancer ou tumeur.

La prothèse humérale 2 est de manière classique réalisée dans un matériau biocompatible, par exemple en alliage chrome-cobalt-molybdène, en acier austénitique ou en alliage de titane.

La prothèse humérale 2 comprend tout d'abord une partie distale 4 comprenant des moyens de fixation à un humérus réséqué. Cette partie a pour vocation de créer une liaison avec un humérus réséqué et donc former une base d'ancrage pour les autres parties de la prothèse humérale 2.

La partie distale 4 peut être formée d'une tige 6 comprenant des moyens de fixation à une partie diaphysaire 22 (décrite ci-après) et destinée à s'étendre à l'intérieur d'une cavité médullaire d'un humérus réséqué. Comme expliqué plus bas, les moyens de fixation peuvent être formés par un premier plot de montage 28.

La tige 6 permet donc un ancrage de la prothèse humérale à l'intérieur de l'humérus réséqué. Cela est possible car elle s'étend sur une longueur assez importante dans l'humérus réséqué. En complément de cette extension, la tige 6 peut comprendre des éléments supplémentaires permettant un ancrage de la partie distale 4 dans l'humérus réséqué. Comme cela est visible sur les figures, la tige 6 peut comprendre des orifices d'ancrage 8 destinés à être traversés par des vis de fixation et ce afin d'ancrer au mieux la tige 6 dans l'os. Alternativement, la tige 6 pourrait être revêtue d'un matériau et/ou comprendre des formes à sa surface permettant un ancrage par coopération avec l'os du patient.

La partie distale 4 peut comprendre une bride 10 placée sur la tige 6 et destinée à entourer au moins partiellement une extrémité d'un humérus réséqué. En d'autres termes, la bride 10 s'étend autour de l'os tandis que la tige 6 s'étend à l'intérieur de l'os et ce afin de stabiliser la partie distale 4, et donc la prothèse humérale 2, dans l'humérus réséqué.

A des fins de stabilisation, il est possible de choisir une bride 10 dont les dimensions permettent un contact surfacique entre elle et la surface externe de l'humérus réséqué.

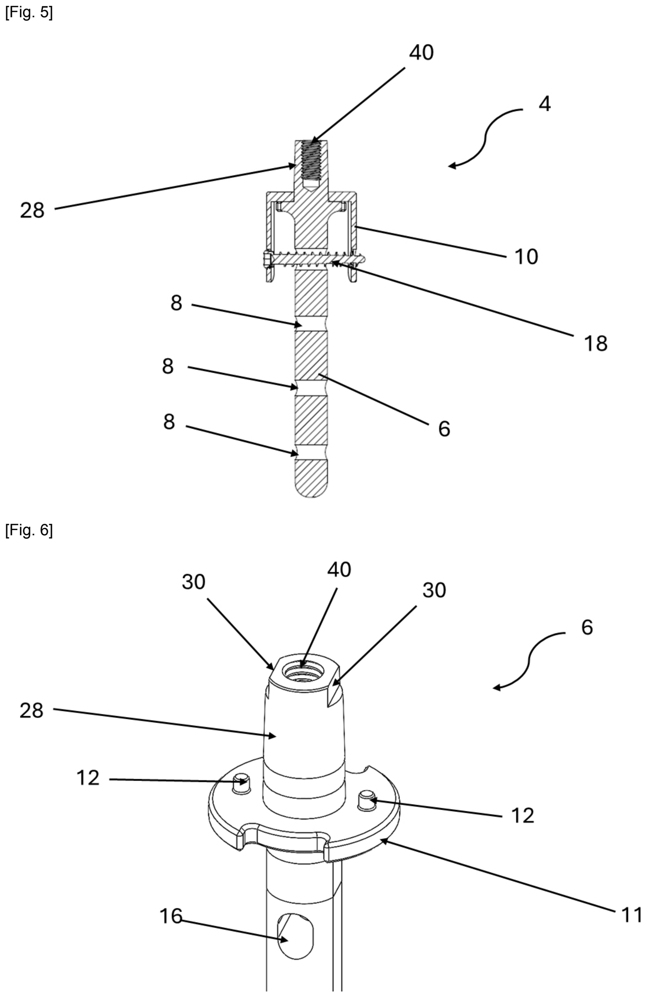

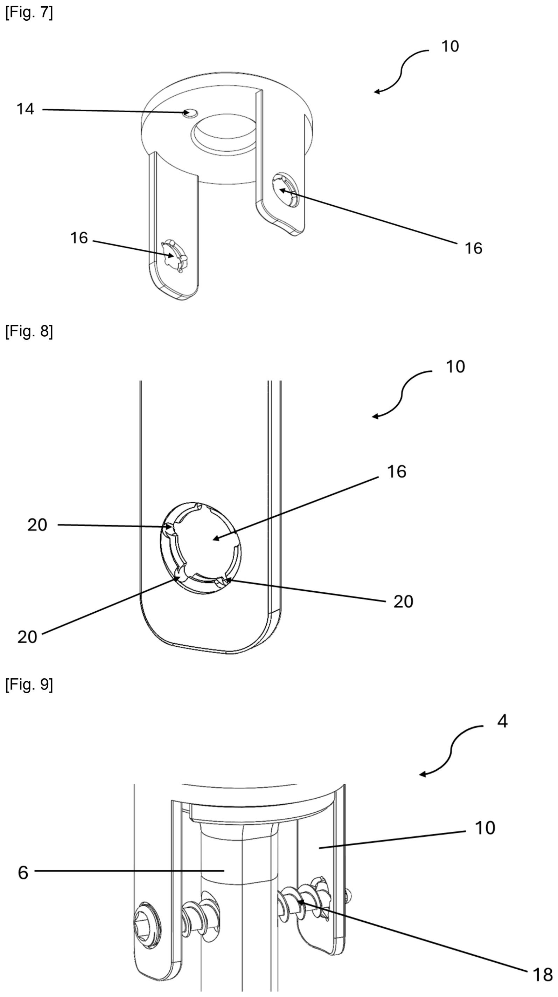

La bride 10 et la tige 6 peuvent comprendre des premiers moyens d'indexage de la bride 10 sur la tige 6. Comme cela est visible sur les figures 6 et 7, la tige peut comprendre un épaulement 11 portant des cônes morses 12 insérés dans des premiers orifices d'indexage 14 lorsque la bride 10 est placée sur la tige 6. Les moyens d'indexage peuvent bien évidemment être de forme différentes et/ou leur nombre peut varier.

La bride 10 et la tige 6 peuvent comprendre des orifices de verrouillage 16 de la partie distale 4 à un humérus, les orifices de verrouillage 16 étant destinés à être alignés et traversés par une vis de verrouillage 18. En d'autres termes, la tige comprend au moins un orifice de verrouillage 16 présent à l'intérieur de la cavité médullaire et la bride 10 comprend, au niveau de sa ou ses extensions entourant l'extrémité de l'humérus réséqué, un ou des orifices de verrouillage 16. Lors de l'implantation de la partie distale 4, ces orifices de verrouillage 16 sont alignés de sorte à utiliser une vis de verrouillage 18 (visible sur la figure 9 par exemple) qui va traverser l'humérus de part en part en passant au travers des orifices de verrouillage 16 et ce afin de créer un point de verrouillage supplémentaire de la partie distale 4 dans l'os. La vis de verrouillage 18 peut être une vis de compression à l'os ou une vis verrouillée sans compression.

Il est possible de créer plusieurs couples d'orifices de verrouillage 16 à différentes hauteurs de la tige 6 et de la bride 10.

Avantageusement, l'orifice de verrouillage 16 de la tige 6 est de forme oblong. Cela permet d'avoir une tolérance de montage tout en bloquant la partie distale 4 en rotation. Un blocage en translation peut être assuré par un autre trou cylindrique présent au niveau de la partie diaphysaire 22.

Avantageusement, le ou les orifices de verrouillage 16 de la bride comprennent un filetage interrompu par des encoches 20. Ces encoches 20 sont visibles sur la figure 8 (cinq encoches sur la figure 8). Ces encoches peuvent être comblées par des tissus biologiques de manière à bloquer la vis de verrouillage 18.

Comme cela est compréhensible au travers de la description ci-dessus, la mise en place et le verrouillage de la partie distale 4 sur l'humérus réséqué sont indépendants du reste de la prothèse humérale 2.

La prothèse humérale 2 comprend en outre une partie diaphysaire 22. Cette partie diaphysaire 22 forme le corps principal de la prothèse humérale 2 remplaçant en grande partie la partie diaphysaire de l'humérus réséqué. Elle s'étend entre la partie distale 4 et une partie métaphysaire 24 décrite ci-après en étant fixée à ces dernières.

La partie diaphysaire 22 est d'un diamètre ainsi que d'une longueur choisie en fonction de l'os du patient, tant sur le diamètre de l'humérus réséqué que sur la longueur de la partie réséquée à reconstruire.

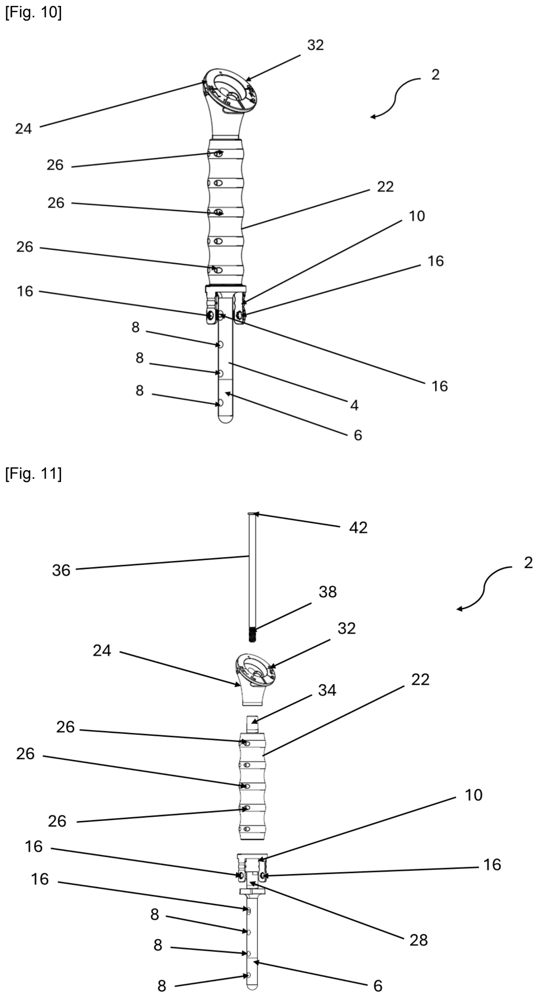

Avantageusement, la partie diaphysaire 22 peut comprendre des trous de suture 26 permettant la suture du muscle deltoïde, et plus généralement des tissus mous à la partie diaphysaire 22. Les figures 10 et 11 illustrent un second mode de réalisation de la prothèse humérale selon l'invention. Dans ce mode de réalisation, les faces externes de la partie diaphysaire 22 et de la bride 10 ne sont pas lisses mais de largeur variable, cette variation de largeur permettant le placement de fils de suture de tissus mous.

La partie diaphysaire 22 peut comprendre un premier orifice de montage sur la partie distale 4 coopérant avec un premier plot de montage 28 (visible sur les figures 2 à 6) porté par la partie distale 4. Avantageusement, le premier orifice de montage et le premier plot de montage 28 comprennent des deuxièmes moyens d'indexage de la partie diaphysaire 22 sur la partie distale 4. La figure 6 illustre un exemple d'indexage avec la présence de méplats 30 sur le premier plot de montage 28 coopérant avec des épaulements présents au niveau du premier orifice de montage. On peut également constater que la bride 10 est montée sur la tige 6 en étant disposée autour du premier plot de montage 28 et ce grâce à la présente d'un trou traversant ménagé au niveau de la bride 10.

La prothèse humérale 2 comprend également une partie métaphysaire 24 comprenant des moyens de fixation d'un élément articulaire d'une articulation gléno-humérale. En d'autres termes, la partie métaphysaire 24, qui forme la partie la plus proximale de la prothèse humérale 2, forme une partie de réception d'une partie de l'articulation à former avec la glène. L'élément articulaire d'une articulation gléno-humérale est choisi parmi une cupule (dans le cas d'une mise en place d'une prothèse articulaire inversée avec mise en place d'une glénosphère au niveau de la glène) ou une tête humérale (dans le cas d'une mise en place d'une prothèse articulaire anatomique avec mise en place d'un implant glénoïdien concave au niveau de la glène). Le choix de l'élément articulaire de l'articulation gléno-humérale est réalisé fonction du type de prothèse articulaire lui-même choisi en fonction du patient.

Avantageusement, la partie métaphysaire 24 comprend une extrémité proximale plane 32 configurée pour recevoir élément articulaire d'une articulation gléno-humérale. L'extrémité proximale plane 32 peut s'étendre dans un plan incliné par rapport à un axe d'extension de la partie diaphysaire 22 selon un angle compris entre 30° et 60°, de préférence compris entre 40° et 50°, de préférence sensiblement égal à 45°.

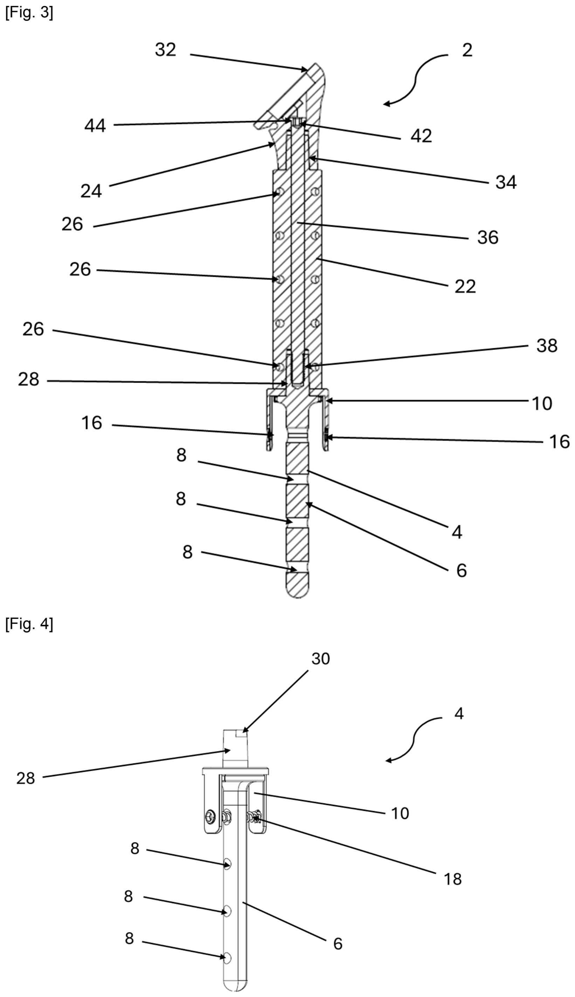

Avantageusement, la partie métaphysaire 24 comprend un deuxième orifice de montage sur la partie diaphysaire 22 coopérant avec un deuxième plot de montage 34 (visible sur les figures 2 et 3) porté par la partie diaphysaire 22.

Contrairement à ce qui a été décrit pour la relation entre la partie distale 4 et la partie diaphysaire 22, la partie métaphysaire 24 peut être orientable en rotation sur la partie diaphysaire 22 lors du montage de la partie métaphysaire 24 sur la partie diaphysaire 22. En d'autres termes, il est possible de ne pas retrouver d'élément d'indexage entre ces deux parties de manière à orienter la partie métaphysaire 24 en fonction du patient.

Avantageusement, la prothèse humérale 2 comprend une vis de blocage 36 s'étendant depuis la partie métaphysaire 24 jusqu'à la partie distale 4 et verrouillant les parties distale 4, diaphysaire 22 et métaphysaire 24 entre elles. Ainsi, un seul élément traversant toutes les parties de la prothèse humérale 2 permet le blocage de toutes les parties entre elles.

La vis de blocage 36 peut comprendre une extrémité filetée 38 (visible sur la figure 2) vissée dans un orifice fileté 40 (visible sur les figures 5 et 6) ménagé au niveau de la partie distale 4. Elle peut également comprendre une butée 42 en appui contre un épaulement 44 de la partie métaphysaire 24. La fixation peut dès lors être effectuée par vissage jusqu'à ce que la butée 42 arrive en appui contre l'épaulement 44. En cas d'absence de butée 42 et d'épaulement 44, il est possible que l'orifice fileté 40 soit un trou borgne au fond duquel l'extrémité filetée 38 de la vis de blocage 36 arrive en butée.

L'invention a également pour objet un kit d'implantation pour la conception d'une prothèse humérale 2 telle que décrite ci-dessus, le kit comprenant :
- Plusieurs parties distales 4 de longueur et/ou largeur différentes,
- Plusieurs parties métaphysaires 24 de longueur et/ou largeur différentes, et
- Plusieurs parties diaphysaires 22 de longueur et/ou largeur différentes.

Un tel kit permet donc de choisir des parties ayant des propriétés dimensionnelles particulières pour créer une prothèse humérale 2 adaptée à chaque patient.

Il est possible, dans l'optique de la sélection de parties de prothèse humérale 2 adaptées à un patient, que :
- La bride 10 et/ou la tige 6 comprennent un nombre et/ou un positionnement différents d'orifices de verrouillage 16 d'une partie distale 4 à une autre.
- La bride 10 soit d'une longueur différente d'une partie distale 4 à une autre.
- La tige 6 soit d'une longueur différente d'une partie distale 4 à une autre.
- La tige 6 comprenne un nombre et/ou un positionnement différents d'orifices d'ancrage 8 d'une partie distale 4 à une autre.
- La partie diaphysaire 22 comprenne un nombre et/ou un positionnement différents de trous de suture 26 d'une partie diaphysaire 22 à une autre.
- L'extrémité proximale plane 32 s'étende dans un plan dont l'inclinaison par rapport à un axe d'extension de la partie diaphysaire 22 différent d'une partie métaphysaire 24 à une autre.

### Liste de références

- 2 :: prothèse humérale
- 4 :: partie distale
- 6 :: tige
- 8 :: orifices d'ancrage
- 10 :: bride
- 11 :: premier épaulement
- 12 :: cônes morses
- 14 :: premiers orifices d'indexage
- 16 :: orifices de verrouillage
- 18 :: vis de verrouillage
- 20 :: encoches
- 22 :: partie diaphysaire
- 24 :: partie métaphysaire
- 26 :: trous de suture
- 28 :: premier plot de montage
- 30 :: méplats
- 32 :: extrémité proximale plane
- 34 :: deuxième plot de montage
- 36 :: vis de blocage
- 38 :: extrémité filetée
- 40 :: orifice fileté
- 42 :: butée
- 44 :: épaulement

## Revendications

1. Prothèse humérale (2) comprenant :
- une partie distale (4) comprenant des moyens de fixation à un humérus réséqué,
- une partie métaphysaire (24) comprenant des moyens de fixation d'un élément articulaire d'une articulation gléno-humérale,
- une partie diaphysaire (22) configurée pour remplacer une partie d'humérus réséquée, s'étendant entre la partie distale (4) et la partie métaphysaire (24) et étant fixée à la partie distale (4) et à la partie métaphysaire (24).

2. Prothèse humérale (2) selon la revendication 1, dans laquelle la partie distale (4) comprend une tige (6) comprenant des moyens de fixation à la partie diaphysaire (22) et destinée à s'étendre à l'intérieur d'une cavité médullaire d'un humérus réséqué.

3. Prothèse humérale (2) selon la revendication 2, dans laquelle la partie distale (4) comprend une bride (10) placée sur la tige (6) et destinée à entourer au moins partiellement une extrémité d'un humérus réséqué.

4. Prothèse humérale (2) selon la revendication 3, dans laquelle la bride (10) et la tige (6) comprennent des premiers moyens d'indexage de la bride (10) sur la tige (6).

5. Prothèse humérale (2) selon l'une quelconque des revendications 3 ou 4, dans laquelle la bride (10) et la tige (6) comprennent des orifices de verrouillage (16) de la partie distale (4) à un humérus, les orifices de verrouillage (16) étant destinés à être alignés et traversés par une vis de verrouillage (18) lors du montage de la partie distale sur un humérus réséqué,

6. Prothèse humérale (2) selon l'une quelconque des revendications précédentes, dans laquelle la partie diaphysaire (22) comprend un premier orifice de montage sur la partie distale (4) coopérant avec un premier plot de montage (28) porté par la partie distale (4).

7. Prothèse humérale (2) selon la revendication 6, dans laquelle l'orifice de montage et le plot de montage comprennent des deuxièmes moyens d'indexage de la partie diaphysaire (22) sur la partie distale (4).

8. Prothèse humérale (2) selon l'une quelconque des revendications précédentes, dans laquelle la partie métaphysaire (24) comprend un deuxième orifice de montage sur la partie diaphysaire coopérant avec un deuxième plot de montage (34) porté par la partie diaphysaire (22).

9. Prothèse humérale (2) selon l'une quelconque des revendications précédentes, dans laquelle la partie métaphysaire (24) est orientable en rotation sur la partie diaphysaire (22) lors du montage de la partie métaphysaire (24) sur la partie diaphysaire (22).

10. Prothèse humérale (2) selon l'une quelconque des revendications précédentes, dans laquelle la partie métaphysaire (24) comprend une extrémité proximale plane (32) configurée pour recevoir élément articulaire d'une articulation gléno-humérale.

11. Prothèse humérale (2) selon l'une quelconque des revendications précédentes, comprenant une vis de blocage (36) s'étendant depuis la partie métaphysaire (24) jusqu'à la partie distale (4) et verrouille les parties distale (4), diaphysaire (22) et métaphysaire (24) entre elles.

12. Prothèse humérale (2) selon la revendication 11, dans laquelle la vis de blocage (36) comprend une extrémité filetée (38) vissée dans un orifice fileté (40) ménagé au niveau de la partie distale (4).

13. Prothèse humérale (2) selon l'une quelconque des revendications 11 ou 12, dans laquelle la vis de blocage (38) comprend une butée (42) en appui contre un épaulement (44) de la partie métaphysaire (24).

14. Kit d'implantation pour la conception d'une prothèse humérale (2) selon l'une quelconque des revendications précédentes, le kit comprenant :
- Plusieurs parties distales (4) de longueur et/ou largeur différentes,
- Plusieurs parties métaphysaires (24) de longueur et/ou largeur différentes, et
- Plusieurs parties diaphysaires (22) de longueur et/ou largeur différentes.
